# FASCICULE DE BREVET EUROPEEN

(11) **EP 0 874 687 B1**
(45) Date de publication et mention de la délivrance du brevet: **03.07.2002**
(21) Numéro de dépôt: 96938279.5
(22) Date de dépôt: 08.11.1996
(51) Int. Cl.: B01J 23/62, C07C 45/51

(54) **PROCEDE DE PREPARATION D'UN CATALYSEUR BI-METALLIQUE RUTHENIUM/ETAIN**
VERFAHREN ZUR HERSTELLUNG EINES BIMETTALLISCHE RUTHENIUM/ZINN KATALYSATOR
METHOD FOR PREPARING A BIMETALLIC RUTHENIUM/TIN CATALYST

(30) Priorité: 08.11.1995 FR 9513185
(43) Date de publication de la demande: 04.11.1998
(73) Titulaire: RHONE-POULENC CHIMIE, 92408 Courbevoie Cédex (FR)
(72) Inventeur: JACQUOT, Roland, F-69110 Sainte-Foy-lès-Lyon (FR)
(74) Mandataire: Dutruc-Rosset, Marie-Claude
(86) Numéro de dépôt international: FR9601762
(87) Numéro de publication internationale: WO9717135

(56) Documents cités:
- EP-A- 0 539 274
- EP-A- 0 626 201
- WO-A-93/14866
- WO-A-96/22832
- US-A- 4 117 082
- US-A- 4 218 401
- US-A- 4 490 482

## Description

La présente invention a pour objet un nouveau procédé de préparation d'un catalyseur bi-métallique ruthénium/étain.

On a décrit dans EP-A-0 539 274, un procédé de préparation d'aldéhydes et de leurs dérivés selon un procédé qui consiste à effectuer la réduction en phase vapeur en présence d'hydrogène, d'acides, d'esters ou d'anhydrides carboxyliques, en présence d'un catalyseur bi-métallique ruthénium/étain.

Bien que des catalyseurs de type ruthénium/étain comprenant du bore conviennent à la mise en oeuvre du procédé décrit, les catalyseurs tout particulièrement intéressants sont les catalyseurs bi-métalliques comprenant de l'étain et du ruthénium, exempt de bore qui comprennent du ruthénium et de l'étain mis en oeuvre en quantités telles que le rapport molaire étain/ruthénium est d'au moins 2, de préférence compris entre 2 et 10 inclus et encore plus préférentiellement entre 2 et 6 inclus.

Il est possible d'utiliser différents types de catalyseurs qui peuvent être ou non supportés.

Généralement, le ruthénium représente entre 0,1 et 50 % du poids du catalyseur.

Dans le cas où l'on fait appel à un catalyseur massique, le ruthénium représente de 10 % à 50 % du poids du catalyseur.

Dans un mode préféré de mise en oeuvre, on utilise toutefois un catalyseur sous forme supportée. A cet effet, le support peut être choisi notamment parmi les oxydes de métaux, tels que les oxydes d'aluminium, de silicium et/ou de zirconium, ou encore parmi les charbons éventuellement activés par un traitement bien connu à l'acide nitrique, le noir d'acétylène, ou les résines.

Si la phase catalytique est déposée sur un support, la teneur en ruthénium du catalyseur est avantageusement choisie entre 0,1 et 20,0 % en poids, et encore plus préférentiellement, entre 0,5 et 3,0 % en poids.

Un mode de préparation desdits catalyseurs décrit dans EP-A-0 539 274 consiste à mélanger le chlorure de ruthénium III et le chlorure d'étain II puis à ajouter le support solide.

L'inconvénient dont souffre ce procédé est qu'il ne permet pas d'obtenir un catalyseur parfaitement homogène susceptible d'être fabriqué à l'échelle industrielle. En effet, le chlorure d'étain II s'hydrolyse partiellement et précipite à la surface du support alors que le chlorure de ruthénium III pénètre dans les pores du support. Il s'ensuit que le support n'est pas imprégné de manière uniforme par les précurseurs des métaux ruthénium et étain et que le catalyseur obtenu n'est pas très homogène.

Un procédé du même type est décrit dans WO-A-96/22832 conduisant à un catalyseur bi-métallique ruthénium/étain comprenant un support et une phase métallique recouvrant au moins en partie ledit support comportant au moins en partie un intermétallique ruthénium-étain au moins partiellement sous forme du composé défini de Ru₃Sn₇

L'objectif de la présente invention est de fournir un procédé de préparation dudit catalyseur permettant d'obvier aux inconvénients précités.

Il a maintenant été trouvé et c'est ce qui constitue l'objet de la présente invention un procédé de préparation d'un catalyseur bi-métallique ruthénium/étain comprenant un support et une phase métallique recouvrant au moins en partie ledit support comportant au moins en partie un intermétallique ruthénium-étain au moins partiellement sous forme du composé défini de Ru₃Sn₇ caractérisé par le fait que qu'il consiste à effectuer la réduction d'un complexe du ruthénium et d'étain ayant une électrovalence - 4 et un nombre de coordination de 6 et dont au moins l'un des coordinats est un halogénure d'étain ; les autres coordinats pouvant être éventuellement constitués par un atome d'halogène.

Selon un mode préféré du procédé de l'invention, on effectue la réduction d'un complexe répondant plus particulièrement à la formule (A) suivante :

[Ru (SnX₃)₆₋ₙXₙ]⁴⁻ (A)

dans ladite formule (A), X représente un atome d'halogène, de préférence, un atome de chlore ou de brome et n est un nombre variant de 0 à 2, et de préférence, égal à 1.

Dans le procédé de l'invention, on fait intervenir préférentiellement les complexes suivants :

- [Ru (SnCl₃)₆]⁴⁻

- [Ru (SnCl₃)₅Cl]⁴⁻

- [Ru (SnCl₃)₄Cl₂]⁴⁻

Il a été trouvé que le catalyseur obtenu était de bonne qualité lorsqu'il était préparé selon le procédé de l'invention tel que précisé.

Intervient donc dans le procédé de l'invention, un complexe halogéné du ruthénium et de l'étain, répondant préférentiellement à la formule (A).

Selon un mode de réalisation préféré de l'invention, on effectue la préparation dudit complexe, par réaction d'un halogénure de ruthénium et d'un halogénure d'étain, en présence d'un acide.

A cet effet, on part d'un halogénure de ruthénium III, de préférence, un chlorure de ruthénium III. Il est également possible de partir d'un sel de ruthénium IV mais il n'y a pas d'avantage supplémentaire d'autant plus qu'il est plus coûteux.

On fait appel donc préférentiellement, à un halogénure de ruthénium III, qui peut être indifféremment sous forme anhydre ou hydratée.

Il est souhaitable que ledit composé ne contienne pas trop d'impuretés. Avantageusement, on a recours à un composé exempt de métaux lourds et présentant une pureté chimique en ruthénium de 99 % par rapport aux autres métaux.

On peut sans inconvénient mettre en oeuvre la forme commerciale du chlorure de ruthénium, RuCl₃, x H₂O comprenant environ de 42 à 43 % en poids de ruthénium.

En ce qui concerne le sel d'étain, on fait appel à un halogénure d'étain dans lequel l'étain présente un degré d'oxydation inférieur à celui du ruthénium.

On met en oeuvre un halogénure d'étain II, de préférence, un chlorure d'étain II.

On peut également mettre en oeuvre le sel sous forme anhydre ou hydratée. Préférentiellement, on utilise également le sel d'étain commercial de formule SnCl₂, 2 H₂O.

Le plus souvent, les halogénures desdits métaux sont mis en oeuvre sous forme de solution aqueuse. La concentration de ces solutions est telle que l'on obtienne une solution homogène susceptible d'être imprégnée sur un support.

Pour ce qui est des quantités engagées des halogénures des métaux précités, elle est déterminée de telle sorte que le rapport entre le nombre de moles d'halogénure de ruthénium et le nombre de moles d'halogénure d'étain varie entre 0,10 et 0,5, et de préférence, entre 0,15 et 0,35. Il est à noter que la limite inférieure ne présente pas de caractère critique car il n'y a pas d'inconvénient à mettre en oeuvre un excès d'halogénure d'étain.

La préparation du complexe par réaction des halogénures de ruthénium et d'étain, s'effectue en présence d'un acide dont la fonction est de solubiliser l'halogénure d'étain et de maintenir soluble le complexe formé.

On peut faire appel à tout acide fort, de préférence, minéral mais l'on préfère utiliser l'hydracide dont l'halogénure est identique à l'halogénure intervenant dans les sels de ruthénium et d'étain.

Ainsi, l'acide chlorhydrique est généralement l'acide préféré.

La quantité d'acide mise en oeuvre est de préférence, d'au moins 1 mole d'acide par mole d'halogénure de ruthénium, et plus particulièrement entre 1 à 5 moles d'acide par mole d'halogénure de ruthénium. La borne supérieure n'est pas critique et peut être dépassée sans inconvénient. La quantité préférée d'acide se situe aux environs de 3 moles d'acide par mole d'halogénure de ruthénium.

D'un point de vue pratique, la préparation du complexe se fait par mélange, dans un ordre quelconque, de l'halogénure de ruthénium (de préférence, le chlorure de ruthénium III) de l'halogénure d'étain (de préférence, le chlorure d'étain II) et de l'acide fort (de préférence, l'acide chlorhydrique).

On porte le mélange réactionnel à une température allant de 20°C à 100°C, de préférence, entre 70°C et 90°C.

La durée de cette opération peut varier largement et l'on précise à titre illustratif, qu'une durée allant de 1 à 3 heures convient tout à fait bien.

Le complexe se forme assez rapidement mais il reste en solution.

Ensuite, si nécessaire, on ramène la température à la température ambiante c'est-à-dire à une température le plus souvent comprise entre 15°C et 25°C.

La préparation du catalyseur peut être faite selon deux modes de réalisation.

Une première variante consiste, lorsque le support se présente sous forme de poudre, tel que par exemple, l'alumine ou le silice, de l'ajouter à la solution du complexe obtenu, à conduire ensuite l'hydrolyse puis à séparer le solide obtenu, de préférence, par filtration et à le malaxer et l'extruder. On obtient ainsi un catalyseur mis en forme.

On effectue l'hydrolyse du complexe obtenu par ajout d'eau.

La quantité d'eau mise en oeuvre n'est pas critique : elle représente généralement de 1 à 100 fois le poids du complexe.

Suite à cette hydrolyse, le complexe précipite sur le support.

Le solide obtenu peut être séparé selon les techniques classiques de séparation solide/liquide, de préférence, par filtration conduite généralement à la température ambiante.

Le solide obtenu est ensuite extrudé.

Selon une autre variante préférée du procédé de l'invention, la solution du complexe obtenu précédemment est utilisée pour imprégner un support.

Le support peut être sous une forme quelconque, par exemple, poudre, billes, granulés, extrudés...

En ce qui concerne la nature du support, des exemples de support sont donnés ci-dessus tels que les oxydes de métaux, tels que les oxydes d'aluminium, de silicium et/ou de zirconium, ou encore parmi les charbons actifs ou les résines.

Dans le cas d'un catalyseur supporté, la teneur en ruthénium est adaptée par l'homme du métier en fonction du support (nature, surface spécifique).

Généralement, la teneur en ruthénium du catalyseur est avantageusement choisie entre 0,1 et 20,0 % en poids, et encore plus préférentiellement, entre 0,5 et 3,0 % en poids.

D'un point de vue pratique, on dépose les métaux sur support en imprégnant ledit support avec la solution du complexe obtenu selon le procédé décrit ci-dessus.

La solution aqueuse d'imprégnation comprend le complexe de ruthénium et d'étain à raison de 1 % à 20 % en poids de ruthénium.

D'une manière pratique, on peut réaliser l'imprégnation en pulvérisant sur le support mis en mouvement, par exemple, par la rotation d'un drageoir, la solution comprenant le complexe de ruthénium et d'étain.

On peut également partir d'un support résultant d'une agglomération de ses particules selon les techniques bien connues, par exemple, d'extrusion ou de pastillage par pression, puis imprégner le support en le plongeant dans la solution dudit complexe.

Selon une variante préférée de l'invention, l'imprégnation est réalisée "à sec" c'est-à-dire le volume total de la solution de complexe utilisé est approximativement égal au volume poreux présenté par le support. Pour la détermination du volume poreux, on peut la faire selon toute technique connue, notamment selon la méthode au porosimètre à mercure (norme ASTM D 4284-83) ou bien mesurer sur un échantillon, la quantité d'eau qu'il absorbe.

Dans une étape suivante, le support imprégné ou bien le précipité séparé obtenu selon le première variante est alors soumis à une opération de réduction.

Une variante préférée de l'invention consiste à faire au préalable une étape de séchage.

Le séchage est effectué le plus souvent à l'air à une température qui peut aller de la température ambiante, par exemple 20°C jusqu'à 100°C.

La durée du séchage est poursuivie jusqu'à obtention d'un poids constant.

Généralement, elle varie de 1 à 24 heures, selon la température choisie.

Dans une étape suivante, on effectue la réduction du complexe en mettant le catalyseur sous forme massique ou supportée en contact avec l'agent réducteur.

Il est possible d'envisager un réducteur chimique mais cela ne présente aucun avantage spécifique. Ainsi, la réduction est conduite préférentiellement avec de l'hydrogène.

L'hydrogène peut être injecté à la pression atmosphérique ou sous une légère pression, par exemple de 0,5 à 10 bar, de préférence, entre 1 et 2 bar.

L'hydrogène peut également être dilué dans un gaz inerte tel que l'azote ou l'hélium.

Avantageusement, la réaction de réduction est conduite à une température d'au moins 350°C, de préférence comprise entre 350°C et 600°C, et encore plus préférentiellement entre 400 et 500°C.

Il est entendu que la réduction peut être également effectuée lors de la mise en oeuvre du catalyseur dans l'hypothèse où il est mis en oeuvre dans une réaction de réduction d'un substrat en présence d'hydrogène.

Ainsi, dans le procédé de préparation d'aldéhydes et de leurs dérivés décrits dans EP-A-0 539 274, selon un procédé qui consiste à effectuer la réduction en phase vapeur en présence d'hydrogène, d'acides, d'esters ou d'anhydrides carboxyliques, en présence d'un catalyseur bi-métallique ruthénium/étain, ledit catalyseur peut être préparé en début de réaction, par réduction d'un complexe de ruthénium et d'étain tel que défini par l'invention.

Le catalyseur supporté de type ruthénium-étain ainsi obtenu est particulièrement homogène et peut être aisément préparé à l'échelle industrielle.

Il comprend une phase métallique recouvrant au moins en partie ledit support comportant au moins en partie un intermétallique ruthénium-étain au moins partiellement sous forme du composé défini de Ru₃Sn₇.

Avantageusement, la phase contenant le ruthénium et l'étain présente un rapport atomique Sn/Ru au moins égal à 2/3, avantageusement à 3/2, de préférence à 7/3.

Par ailleurs, il est préférable que le rapport atomique Sn/Ru soit au plus égal à 3, avantageusement à 5/2.

Dans les catalyseurs préférés, ladite phase recouvrant au moins en partie ledit support contient au moins 50 %, avantageusement 80 %, de préférence au moins 90 % de ladite phase intermétallique.

Enfin il est souhaitable qu'au moins 90 %, avantageusement au moins 95 %, de préférence 98 % du ruthénium présent sur le support soit sous la forme de ladite phase recouvrant ledit support.

Il est très intéressant de mettre en oeuvre le catalyseur obtenu selon l'invention, dans le procédé de préparation d'aldéhydes et dérivés décrits dans EP-A-0 539 274 qui est incorporé par référence dans la présente demande.

En effet, il peut être avantageusement mis en oeuvre pour effectuer la réduction par l'hydrogène, d'acides, d'esters ou d'anhydrides carboxyliques en phase vapeur.

Plus particulièrement, le catalyseur est bien adaptée à la préparation d'aldéhydes de formule générale : 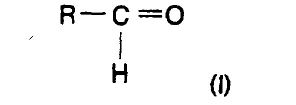 dans laquelle R représente un atome d'hydrogène ou un radical hydrocarboné, éventuellement substitué comportant de 1 à 40 atomes de carbone qui peut être un radical aliphatique acyclique saturé ou insaturé, linéaire ou ramifié ; un radical carbocyclique ou hétérocyclique saturé, insaturé ou aromatique, monocyclique ou polycyclique, par réduction d'esters, d'anhydrides ou d'acides de formule : 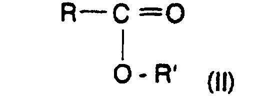 dans laquelle :
- R est défini comme précédemment,
- R' représente :
   - un groupement R tel que précédemment défini,
   - un groupement 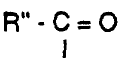 dans lequel R" a la signification donnée pour R,
   - les deux groupements R et R" pouvant être liés entre eux pour former un cycle saturé ou insaturé ayant de 5 à 7 atomes et comprenant la fonction anhydride,
   - les deux groupements R et R" par l'intermédiaire de deux atomes vicinaux pouvant former ensemble un pont d'un système bicyclique ortho-condensé.

Conformément au procédé de l'invention, on peut mettre en oeuvre n'importe quel acide carboxylique susceptible d'être sous forme gazeuse dans les conditions de l'invention.

Le procédé de l'invention s'applique à tout acide carboxylique mono- ou polycarboxylique tels que les acides aliphatiques saturés ou insaturés ; carbocycliques ou hétérocycliques, saturés, insaturés ou aromatiques, monocycliques ou polycycliques ; aliphatiques saturés ou insaturés porteurs d'un substituant cyclique tel qu'un cycle carbocyclique ou hétérocyclique saturé, insaturé ou aromatique.

On peut donc faire appel comme matière première de départ à un acide carboxylique répondant à la formule (II) dans laquelle le reste R représente un radical hydrocarboné, substitué ou non qui peut être un radical aliphatique acyclique saturé ou insaturé, linéaire ou ramifié ; un radical carbocyclique ou hétérocyclique saturé, insaturé ou aromatique, monocyclique ou polycyclique.

Les acides carboxyliques ou dérivés mis en oeuvre préférentiellement répondent à la formule (II) dans laquelle R représente un radical hydrocarboné, éventuellement substitué comportant de 1 à 20 atomes de carbone.

Conviennent tout particulièrement bien à la mise en oeuvre du procédé de l'invention, les acides carboxyliques de formule générale (II) dans laquelle R représente un reste hydrocarboné aromatique éventuellement, substitué, monocyclique ou polycyclique.

N'importe quel substituant peut être présent sur le cycle dans la mesure où il ne gêne pas la réaction de réduction de la fonction carboxylique.

R représente préférentiellement un reste hydrocarboné aromatique, et notamment benzénique répondant à la formule générale (III) : 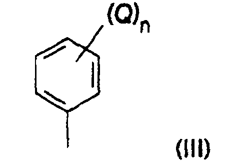 dans ladite formule (III) :
- n est un nombre entier de 0 à 5, de préférence de 0 à 3,
- Q représente R₁, l'un des groupes ou fonctions suivantes :
   . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
   . un radical alcényle linéaire ou ramifié ayant de 2 à 6 atomes de carbone, de préférence, de 2 à 4 atomes de carbone, tel que vinyle, allyle,
   . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
   . un groupe acyle ayant de 2 à 6 atomes de carbone,
   . un radical de formule :

      -R₂-OH

      -R₂-COOR₅

      -R₂-CHO

      -R₂-NO₂

      -R₂-CN

      -R₂-N(R₅)₂

      -R₂-CO-N(R₅)₂

      -R₂-SH

      -R₂-X

      -R₂-CF₃

      dans lesdites formules, R₂ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux R₅, identiques ou différents représentent un atome d'hydrogène ou un radical alkyle linéaire ou ramifié ayant de 1 à 6 atomes de carbone ; X symbolise un atome d'halogène, de préférence un atome de chlore, de brome ou de fluor.
- Q représente R₃ l'un des radicaux plus complexes suivants :
   . un radical 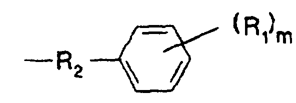 dans lequel R₁ et R₂ ont la signification donnée précédemment et m est un nombre entier de 0 à 5, de préférence de 0 à 3,
   . un radical -R₂-A-R₄ dans lequel R₂ a la signification donnée précédemment, R₄ représente un radical alkyle linéaire ou ramifié ayant de 1 à 6 atomes de carbone, de préférence, de 1 à 4 atomes de carbone ou un radical de formule 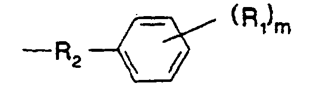 et A symbolise l'un des groupes suivants : 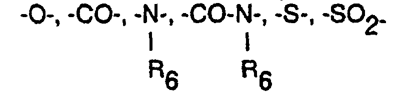 dans ces formules, R₆ représente un atome d'hydrogène ou un radical alkyle linéaire ou ramifié ayant de 1 à 4 atomes de carbone, de préférence, un radical méthyle ou éthyle.

Lorsque n est supérieur à 1, les radicaux Q peuvent être identiques ou différents et 2 atomes de carbone successifs du cycle benzénique peuvent être reliés entre eux par un pont cétalique tels que les radicaux méthylène dioxy ou éthylène dioxy extranucléaires.

De préférence, n est égal à 0, 1, 2 ou 3.

Parmi tous les restes R précités, on met en oeuvre tout préférentiellement dans le procédé de l'invention, les acides carboxyliques ou dérivés répondant à la formule générale (II) dans laquelle R représente un reste aromatique répondant à la formule générale (III) dans laquelle :
- n est égal à 0, 1, 2 ou 3,
- Q représente l'un des groupes ou fonctions suivantes :
   . un atome d'hydrogène,
   . un radical alkyle linéaire ou ramifié, ayant de 1 à 4 atomes de carbone,
   . un radical alkoxy linéaire ou ramifié ayant de 1 à 4 atomes de carbone,
   . un radical méthylène ou éthylène dioxy,
   . un groupe -OH,
   . un groupe -CHO,
   . un groupe NH₂,
   . un radical phényle,
   . un atome d'halogène,
   . un groupe CF₃.

Encore plus préférentiellement, on choisit les composés de formule (II) dans laquelle les radicaux Q identiques ou différents sont un atome d'hydrogène, un groupe hydroxyle, un radical méthyle, un radical méthoxy, un groupe -CHO.

Comme exemples de radicaux R répondant à la formule (III), on peut mentionner plus précisément les radicaux phényle, tolyle ou xylyle et les radicaux biphényle, méthylène-1,1' biphényle, isopropylidène-1,1' biphényle, oxy-1,1' biphényle, imino-1,1' biphényle : lesdits radicaux pouvant être substitués par un ou plusieurs radicaux Q tels que prédéfinis, de préférence un groupe hydroxyle ou un atome d'halogène.

R peut également représenter un reste hydrocarboné aromatique polycyclique ; les cycles pouvant former entre eux des systèmes ortho-condensés, ortho- et péri-condensés. On peut citer plus particulièrement, un reste naphtalénique ; lesdits cycles pouvant être substitués par 1 à 4 radicaux R₁, de préférence 1 à 3, R₁ ayant les significations énoncées précédemment pour les substituants du reste hydrocarboné aromatique de formule générale (III).

Dans la formule générale (II) des acides carboxyliques, R peut représenter également un reste carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué par 1 à 5 radicaux R₁, de préférence 1 à 3, R₁ ayant les significations énoncées précédemment pour les substituants du reste hydrocarboné aromatique de formule générale (III).

Comme exemples préférés de radicaux R, on peut citer les radicaux cyclohexyle ou cyclohexène-yle, éventuellement substitué par des radicaux alkyle linéaires ou ramifiés, ayant de 1 à 4 atomes de carbone.

Comme mentionné précédemment, R peut représenter un reste aliphatique acyclique, saturé ou insaturé, linéaire ou ramifié.

Plus précisément, R représente un reste aliphatique acyclique linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ou des triples liaisons.

La chaîne hydrocarbonée peut être éventuellement :
- interrompue par l'un des groupes suivants : 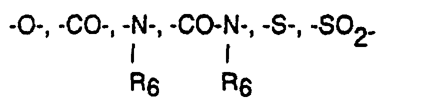 dans ces formules R₆ représente l'hydrogène ou un radical alkyle linéaire ou ramifié ayant de 1 à 4 atomes de carbone, de préférence, un radical méthyle ou éthyle,
- et/ou porteuse de l'un des substituants suivants :
   -OH, -COOR₅, -CHO, -NO₂, -CN, -NH₂, -SH, -X, -CF₃,
   dans ces formules, R₅ ayant la signification donnée précédemment.

Dans un mode préféré de l'invention, R répond à la formule suivante : 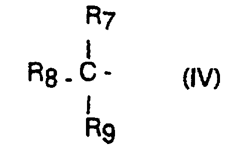 dans laquelle R₇, R₈ et R₉, identiques ou différents, sont choisis parmi un atome d'hydrogène, un radical alkyle linéaire ou ramifié contenant 1 à 10 atomes de carbone, un radical alcényle linéaire ou ramifié contenant 1 à 10 atomes de carbone, un radical alkoxy linéaire ou ramifié contenant 1 à 10 atomes de carbone, un groupe hydroxyle, une fonction amine ou un atome d'halogène ou un groupe -CF₃.

Préférentiellement, R₇ et/ou R₈ et/ou R₉ représentent un groupement insaturé.

Encore plus préférentiellement, dans la formule (IV), l'un des 3 groupements R₇, R₈ et R₉ possède une double liaison conjuguée avec le groupement carbonyle de l'acide, de l'ester ou de l'anhydride carboxylique.

Il est également possible de faire appel à un acide carboxylique ou dérivé de formule (II) dans laquelle R représente un reste aliphatique acyclique, saturé ou insaturé, linéaire ou ramifié pouvant être éventuellement porteur d'un substituant cyclique. Par cycle, on entend un cycle carbocyclique ou hétérocyclique, saturé, insaturé ou aromatique.

Le reste aliphatique acyclique peut être relié au cycle par un lien valentiel ou par l'un des groupes suivants : 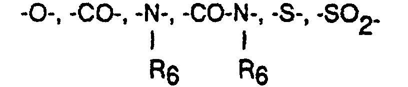 dans ces formules, R₆ ayant la signification donnée précédemment.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs de 1, 2, 3, 4 ou 5 radicaux R₁, identiques ou différents, R₁ ayant les significations énoncées précédemment pour les substituants du reste hydrocarboné aromatique de formule générale (III).

Comme exemples de tels radicaux, on peut mentionner, entre autres, le radical benzyle.

Dans la formule générale (II) des acides carboxyliques, R peut également représenter un reste hétérocyclique, saturé ou non, comportant notamment 5 ou 6 atomes dans le cycle dont 1 ou 2 hétéroatomes tels que les atomes d'azote, de soufre et d'oxygène ; les atomes de carbone de l'hétérocycle pouvant éventuellement être substitués, dans leur totalité ou pour une partie d'entre eux seulement par des radicaux R₁, R₁ ayant les significations énoncées précédemment pour les substituants du reste hydrocarboné aromatique de formule générale (III).

R peut aussi représenter un reste hétérocyclique polycyclique défini comme étant soit un radical constitué par au moins 2 hétérocycles aromatiques ou non contenant au moins un hétéroatome dans chaque cycle et formant entre eux des systèmes ortho- ou ortho- et péri-condensés ou soit un radical constitué par au moins un cycle hydrocarboné aromatique ou non et au moins un hétérocycle aromatique ou non formant entre eux des systèmes ortho- ou ortho- et péri-condensés ; les atomes de carbone desdits cycles pouvant éventuellement être substitués, dans leur totalité ou pour une partie d'entre eux seulement par des radicaux R₁, R₁ ayant les significations énoncées précédemment pour les substituants du reste hydrocarboné aromatique de formule générale (III).

A titre d'exemples de groupements R de type hétérocyclique, on peut citer entre autres, les radicaux furyle, pyrrolyle, thiényle, isoxazolyle, furazannyle, isothiazolyle, imidazolyle, pyrazolyle, pyridyle, pyridazinyle, pyrimidinyle et les radicaux quinolyle, naphtyridinyle, benzofurannyle, indolyle.

Comme exemples d'acides carboxyliques comprenant au moins un groupe carboxylique répondant à la formule (II), on peut mettre en oeuvre plus particulièrement les acides carboxyliques suivants :
- les acides monocarboxyliques aliphatiques saturés tels que l'acide formique, acétique, propionique, butyrique, isobutyrique, valérique, isovalérique, pivalique, laurique, myristique, palmitique, stéarique,
- les acides dicarboxyliques aliphatiques saturés tels que l'acide oxalique, malonique, succinique, glutarique, adipique, pimélique, subérique, azélaïque, sébacique,
- les acides monocarboxyliques ou dicarboxyliques aliphatiques insaturés tels que l'acide acrylique, propiolique, méthacrylique, crotonique, isocrotonique, sénécioïque, acide tiglique, oléïque, maléique, fumarique, citraconique, mésaconique,
- les acides carboxyliques carbocycliques saturés ou insaturés tels que l'acide camphorique, l'acide chrysanthémique,
- les acides carboxyliques hétérocycliques tels que les acides furannecarboxyliques, thiophènecarboxyliques, pyrrolecarboxyliques, pyrazinecarboxyliques, l'acide nicotinique, isonicotinique, l'acide picolinique,
- les acides carboxyliques carbocycliques aromatiques tels que l'acide benzoïque, phtalique, isophtalique, téréphtalique, les acides naphtalènecarboxyliques, les acides toluiques,
- les acides carboxyliques arylaliphatiques saturés tels que, notamment les arylpropioniques comme l'acide 2-phénylpropionique, l'acide 2-[4-(2-butyl-2) phényl]propionique, l'acide 2-(3-benzoylphényl)propionique, l'acide 2-(6-méthoxy-2-naphtyl)propionique ou les acides insaturés comme par exemple l'acide 2-phénylpropénoïque, l'acide cinnamique,
- les acides carboxyliques aliphatiques ou aromatiques halogénés tels que l'acide monofluoroacétique, difluroacétique, monochloroacétique, dichloroacétique, trichloroacétique, monochloropropionique, α-bromopropionique, α-bromobutyrique, trifluoroacétique, l'acide monofluoro-o-benzoïque, l'acide monofluoro-m-benzoïque, l'acide monofluoro-p-benzoïque, l'acide 2,3-difluorobenzoïque, l'acide 2,4-difluorobenzoïque, l'acide 2,5-difluorobenzoïque, l'acide 3,4-difluorobenzoïque, l'acide 2,3,6-trifluorobenzoïque, l'acide 2,4,5-trifluorobenzoïque, l'acide 2,3,4,5 - tétrafluorobenzoïque, l'acide pentafluorobenzoïque, l'acide α,α,α-trifluoro-o-toluique, l'acide α,α,α-trifluoro-m-toluique, l'acide α,α,α-trifluoro-p-toluique, l'acide monochloro-o-benzoïque, l'acide monochloro-m-benzoïque, l'acide monochloro-p-benzoique, l'acide 2,3-dichlorobenzoïque, l'acide 2,4-dichlorobenzoïque, l'acide 2,5-dichlorobenzoïque, l'acide 2,6-dichlorobenzoïque, l'acide 3,4-dichlorobenzoïque, l'acide 3,5-dichlorobenzoïque, l'acide 2,3,5-trichlorobenzoïque, l'acide 2,3,6-trichlorobenzoïque, l'acide 2-chloro-4,5-difluorobenzoïque, l'acide 3-chloro-2,4,5-triflurobenzoïque, l'acide monobromo-o-benzoïque, l'acide monobromo-m-benzoïque, l'acide monobromo-p-benzoïque.
- les hydroxy-acides aliphatiques, cycloaliphatiques, arylaliphatiques, tels que l'acide glycolique, l'acide lactique, l'acide glycérique, l'acide 2-hydroxybutanoïque, l'acide 3-hydroxybutanoïque, l'acide 2-méthyllactique, l'acide 2-hydroxy-4-méthylthiobutanoïque, l'acide tartronique, l'acide malique, l'acide tartrique, l'acide 1-hydroxycyclopropane carboxylique, l'acide 2-hydroxyphénylpropanoïque, l'acide 2-hydroxycinnamique, l'acide 3-hydroxycinnamique, l'acide 4-hydroxycinnamique,
- les acides hydroxybenzoïques suivants : l'acide 2-hydroxybenzoïque (acide salicylique), l'acide 3-hydroxybenzoïque, l'acide 4-hydroxybenzoïque, l'acide 3-méthylsalicylique, l'acide 4-méthylsalicylique, l'acide 5-méthylsalicylique, l'acide 3-hydroxy-4-méthylbenzoïque, l'acide 3-méthoxysalicylique, l'acide 4-méthoxysalicylique, l'acide 5-méthoxysalicylique, l'acide 3-hydroxy-4-méthoxybenzoïque (acide isovanillique), l'acide 4-hydroxy-3-méthoxybenzoïque (acide vanillique), l'acide 3-hydroxy-4,5-diméthoxybenzoïque, l'acide 4-hydroxy-3,5-diméthoxybenzoïque (acide syringique), l'acide 5-hydroxyisophtalique, l'acide 3-aminosalicylique, l'acide 4-aminosalicylique, l'acide 5-aminosalicylique, l'acide 3-hydroxy-2-aminobenzoïque, l'acide 3-nitrosalicylique, l'acide 3-hydroxy-4-nitrobenzoïque, l'acide 4-hydroxy-3-nitrobenzoique, l'acide 3-hydroxy-4-méthyl-2-nitrobenzoïque, l'acide 3,5-diiodosalicylique, l'acide 2,3-dihydroxybenzoïque, l'acide 2,4-dihydroxybenzoïque, l'acide 2,5-dihydroxybenzoïque, l'acide 2,6-dihydroxybenzoïque, l'acide 3,4-dihydroxybenzoïque (acide protocatéchique), l'acide 3,5-dihydroxybenzoïque, l'acide 3,5-dihydroxy-4-méthylbenzoïque, l'acide 2,3,4-trihydroxybenzoïque, l'acide 2,4,6-trihydroxybenzoique, l'acide 3,4,5-trihydroxybenzoïque,
- les alcoxy- et phénoxyacides tels que l'acide méthoxyacétique, phénoxyacétique, 2,4-dichlorophénoxyacétique, phénoxypropionique, 2,4-dichlorophénoxypropionique, p-hydroxyphénoxypropionique, m-chlorophénoxypropionique, l'acide 4-phénoxybenzoïque, l'acide (4-carboxy-4-phénoxy)benzoïque, l'acide pipéronylique,
- les oxo-acides tels que l'acide 2-acétylbenzoïque, l'acide 4-acétylbenzoïque, l'acide 2-benzoylbenzoïque, l'acide 4-benzoylbenzoïque,
- les acyloxy-acides tels que l'acide 3-benzoyloxypropionique, l'acide 2-acétoxybenzoïque, l'acide 4-acétoxybenzoïque,
- les amido-acides tels que l'acide 2-acétamidoacrylique, l'acide 2-acétamidobenzoique, l'acide 3-acétamidobenzoïque, l'acide 4-acétamidobenzoïque,
- les acides aminés éventuellement N-protégés par un groupe protecteur comme par exemple les groupes suivants acyle (acétyle, benzoyle), BOC (butyloxycarbonyl), CBZ (carbobenzoxy), FMOC (fluorényl-9 méthoxycarbonyl), MSOC (méthanesulfényl-2 éthoxycarbonyl).

On peut citer les acides aminés suivants :
- acides aminés aliphatiques : glycine, alanine, valine, leucine, isoleucine,
- acides aminés hydroxylés : sérine, thréonine,
- acides aminés soufrés : cystéine, méthionine,
- acides aminés dicarboxyliques et leurs amides : acide aspartique, asparagine, acide glutamique, glutamine,
   . acides aminés possédant deux groupements basiques : lysine, arginine, histidine,
   . acides aminés aromatiques : phénylalanine, tyrosine, tryptophanne,
   . imino-acides : proline, hydroxyproline.

Les acides mis en oeuvre préférentiellement sont plus particulièrement des acides carboxyliques tels que l'acide benzoïque, l'acide 3,4-difluorobenzoïque, l'acide 4-chlorobenzoïque, l'acide 4-trifluorométhylbenzoïque, l'acide salicylique, l'acide 3-hydroxybenzoïque, l'acide 4-hydroxybenzoïque, l'acide vanillique, l'acide 3,4-diméthoxybenzoïque, l'acide 4-méthoxybenzoïque, l'acide 3,4-dioxyméthylènebenzoïque, l'acide cinnamique, l'acide 6-méthoxy-2-naphtalènecarboxylique, l'acide 6-hydroxy-2-naphtalènecarboxylique, l'acide acétique, l'acide trifluoroacétique, l'acide 2-méthylbutyrique, les acides gras aliphatiques saturés ou non ayant de 6 à 20 atomes de carbone, de préférence, l'acide heptanoïque, l'acide nonanoïque, l'acide undécanoïque, l'acide oleïque, l'acide heptadécanoïque, l'acide stéarique, l'acide laurique, l'acide undécènoïque, l'acide 2-méthylnonanoïque, l'acide 3,7-diméthyl-2,6-octadiènecarboxylique, l'acide sénécioïque, l'acide cyclohexaneoïque.

Conformément à la présente invention, on peut faire appel à un acide carboxylique sous la forme de son anhydride.

Comme exemples d'anhydrides carboxyliques, on peut mentionner plus particulièrement les anhydrides des acides carboxyliques précités et les anhydrides cycliques.

En effet, lorsque l'anhydride répond à la formule (II) dans laquelle R' est un groupement 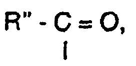 les deux groupements R et R" peuvent être liés entre eux pour former un cycle saturé ou insaturé ayant de 5 à 7 atomes comprenant la fonction anhydride. Ils forment de préférence un radical alkylène linéaire ou ramifié ayant de 2 à 6 atomes de carbone et encore plus préférentiellement un radical -(CH₂)ₜ- avec t égal de 2 à 4.

Comme exemples de tels anhydrides cycliques, on peut citer l'anhydride succinique ou l'anhydride maléique.

Lorsque l'anhydride répond à la formule (II) dans laquelle R' est un groupement 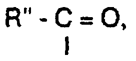 les deux groupements R et R", par l'intermédiaire de deux atomes vicinaux peuvent former ensemble un pont d'un système bicyclique orthocondensé.

Les composés préférés sont bicycliques et constitués d'un cycle benzénique et d'un hétérocycle puisque le cycle comprend l'atome d'oxygène de la fonction anhydride, ledit cycle ayant de préférence de 5 à 6 atomes. Comme exemples de tels anhydrides cycliques d'acides polycarboxyliques, on peut mentionner l'anhydride phtalique.

Le procédé de l'invention est mis en oeuvre en phase gazeuse.

Avantageusement, la réaction est conduite à une température comprise entre 100°C et 500°C, et encore plus préférentiellement entre 200 et 400°C. Il est entendu que la température est adaptée par l'homme du métier en fonction de l'acide de départ, et de la vitesse de réaction recherchée.

Par ailleurs, il peut être particulièrement avantageux de procéder à une activation préalable du catalyseur, par forte élévation de température. Notamment, le catalyseur peut être préalablement soumis à des températures proches de 500°C environ, et préférentiellement de 450°C. L'activation est conduite avantageusement sous courant d'hydrogène.

Une manière pratique de mettre en oeuvre la présente invention consiste à introduire dans un réacteur une quantité désirée de catalyseur, éventuellement entre 2 lits de quartz pour favoriser la mise en contact des réactifs. La température du réacteur est ensuite élevée sous courant d'hydrogène jusqu'à une valeur déterminée, permettant d'activer le catalyseur, puis ramenée à la température de réaction. L'acide est ensuite injecté au débit souhaité et l'aldéhyde formé est récupéré.

Préférentiellement, l'acide est injecté directement sous forme gazeuse après avoir été vaporisé par chauffage.

Toutefois, il peut également être injecté en solution dans un solvant inerte pour la réaction. On peut citer en particulier comme solvants inertes les hydrocarbures aliphatiques (par exemple l'hexane), alicycliques (par exemple le cyclohexane), aromatiques (par exemple le toluène), ou les éthers (par exemple le diméthoxyéthane).

Sous l'effet de la température élevée, l'acide ainsi injecté est vaporisé au niveau du premier lit de quartz. L'hydrogène peut être injecté à la pression atmosphérique ou sous une légère pression compatible avec la phase vapeur (quelques bar, par exemple de 0,5 à 10 bar). L'hydrogène peut également être dilué dans un gaz inerte tel que l'azote ou l'hélium.

Avantageusement, pour 1 ml de catalyseur, l'hydrogène est injecté à un débit compris entre 0,1 et 10 litres par heure, et l'acide à un débit liquide au plus égal à 10 ml/h, et de préférence compris entre 0,5 et 5 ml/h.

En fin de réaction, on récupère l'aldéhyde par tout moyen approprié tel que distillation ou cristallisation. Dans certains cas, notamment dans le cas du fluoral, l'aldéhyde peut être obtenu sous une forme hydratée.

Le catalyseur obtenu selon le procédé de l'invention peut être mis en oeuvre dans un procédé de préparation de nombreuses aldéhydes qui sont utilisés comme produits intermédiaires pharmaceutiques et/ou agrochimiques tels que par exemple, le 3,4-difluorobenzaldéhyde, le 4-chlorobenzaldéhyde.

Il est particulièrement intéressant pour la préparation de l'aldéhyde salicylique qui peut être utilisé, entre autres, pour la préparation de la coumarine : celle-ci résultant d'une étape de cyclisation bien connue et largement décrite dans la littérature (KIRK-OTHMER - Encyclopedia of Chemical Technology 7, p. 198, 3^{ème} édition).

Le catalyseur obtenu selon l'invention convient également pour la préparation d'autres aldéhydes aromatiques tels que le 3-hydroxybenzaldéhyde, le 4-hydroxybenzaldéhyde, la vanilline, le vératraldéhyde, le p-anisaldéhyde, le pipéronal, l'aldéhyde cinnamique.

La présente invention est également utilisable pour la synthèse d'aldéhydes divers. Elle peut servir pour préparer des aldéhydes saturés tels que le fluoral ou l'aldéhyde acétique. Elle est particulièrement adaptée à la synthèse d'aldéhydes insaturés, notamment dans la chimie des terpènes (prénal, citral ...), intermédiaires de synthèse de vitamines A ou E.

On ne sortira du cadre de la présente invention à fabriquer selon le procédé de l'invention, les aldéhydes sous la forme de leurs dérivés tels que leurs acétals ou leurs hémiacétals, par réaction de l'aldéhyde et d'un alcool qui est introduit soit conjointement à l'acide, soit en fin de réaction. Comme exemples d'alcools classiquement utilisés, on peut citer le méthanol ou l'éthanol.

On donne ci-après des exemples de réalisation de l'invention qui sont donnés à titre illustratif et sans caractère limitatif.

### Exemple 1

### Préparation du catalyseur imprégné à sec.

Dans un tricol, on introduit 1,92 g de RuCl₃, x H₂O comprenant 42 % en poids de ruthénium et 10,7 g de SnCl₂, 2H₂O et 13 ml d'une solution aqueuse d'acide chlorhydrique 3N.

On chauffe en agitant à 90°C.

On maintient pendant 1 heure à cette température.

On refroidit ensuite cette solution.

On imprègne 40 g de billes d'alumine α (surface spécifique = 5 - 10 m²/g et volume poreux = 44 - 54 cm³ pour 100 g) commercialisées par Rhône-Poulenc sous la dénomination Sphéralite 512 ayant un diamètre de billes de 2 à 4 mm.

On sèche ensuite les billes imprégnées dans une étuve ventilée, jusqu'à obtention d'un poids constant.

Le catalyseur est ensuite traité à 450°C sous courant d'hydrogène, pendant 4 heures.

### Exemple 2

### Préparation du catalyseur à partir d'un support sous forme de poudre.

Dans un tricol, on introduit 1,92 g de RuCl₃, x H₂O comprenant 42 % en poids de ruthénium et 10,7 g de SnCl₂, 2H₂O et 13 ml d'une solution aqueuse d'acide chlorhydrique 3N.

On chauffe en agitant à 90°C pendant 1 heure.

On refroidit ensuite cette solution à 20°C.

On ajoute ensuite 80 g de silice Degussa OX 50 (surface spécifique = 50 m²/g et taille moyenne des particules primaires de 40 nm) et 4000 ml d'eau.

Le précipité est filtré, lavé à l'eau.

Le gâteau est ensuite malaxé et extrudé.

Les extrudés sont ensuite séchés à l'air, jusqu'à obtention d'un poids constant

Le catalyseur est ensuite traité par l'hydrogène à 450°C sous courant d'hydrogène, pendant 4 heures.

### Exemple 3

### Hydrogénation de l'acide trifluoroacétique.

Dans un réacteur tubulaire en nickel de diamètre 2,54 cm, on introduit 60 g de catalyseur préparé selon l'exemple 1.

On traite celui-ci sous courant d'hydrogène de 11 litres par heure en chauffant à 450°C.

On maintient ces conditions, 15 heures.

On ramène la température à 320°C et on injecte l'acide trifluoroacétique à raison de 20 g/h.

Le taux de transformation est de 80 % et le rendement en fluoral hydraté est de 70 %.

Après 300 heures, les performances du catalyseur sont identiques.

## Revendications

1. Procédé de préparation d'un catalyseur bi-métallique ruthénium/étain comprenant un support et une phase métallique recouvrant au moins en partie ledit support comportant au moins en partie un intermétallique ruthénium-étain au moins partiellement sous forme du composé défini de Ru₃Sn₇ **caractérisé par le fait que** qu'il consiste à effectuer la réduction d'un complexe du ruthénium et d'étain ayant une électrovalence - 4 et un nombre de coordination de 6 et dont au moins l'un des coordinats est un halogénure d'étain ; les autres coordinats pouvant être éventuellement constitués par un atome d'halogène.

2. Procédé selon la revendication 1 **caractérisé par le fait que** le complexe répond à la formule (A) suivante :
[Ru (SnX₃)₆₋ₙXₙ]⁴⁻ (A)
dans ladite formule (A), X représente un atome d'halogène, de préférence, un atome de chlore ou de brome et n est un nombre variant de 0 à 2, et de préférence, égal à 1.

3. Procédé selon la revendication 2 **caractérisé par le fait que** le complexe répond à la formule (A) suivante :
- [Ru (SnCl₃)₆]⁴⁻
- [Ru (SnCl₃)₅Cl]⁴⁻
- [Ru (SnCl₃)₄Cl₂]⁴⁻.

4. Procédé selon l'une des revendications 1 à 3 **caractérisé par le fait que** l'on prépare le complexe par réaction d'un halogénure de ruthénium et d'un halogénure d'étain, en présence d'un acide.

5. Procédé selon la revendication 4 **caractérisé par le fait que** l'halogénure de ruthénium est un halogénure de ruthénium III, sous forme anhydre ou hydratée, de préférence, un chlorure de ruthénium III, et l'halogénure d'étain II, sous forme anhydre ou hydratée, est de préférence, le chlorure d'étain II.

6. Procédé selon l'une des revendications 4 et 5 **caractérisé par le fait que** le rapport entre le nombre de moles d'halogénure de ruthénium et le nombre de moles d'halogénure d'étain varie entre 0,10 et 0,5, et de préférence, entre 0,15 et 0,35.

7. Procédé selon l'une des revendications 4 à 6 **caractérisé par le fait que** l'acide est tout acide fort minéral, de préférence, l'hydracide dont l'halogénure est identique à l'halogénure intervenant dans les sels de ruthénium et d'étain.

8. Procédé selon l'une des revendications 4 à 7 **caractérisé par le fait que** la quantité d'acide mise en oeuvre est de préférence, d'au moins 1 mole d'acide par mole d'halogénure de ruthénium, plus préférentiellement, entre 1 à 5 moles d'acide par mole d'halogénure de ruthénium et encore plus préférentiellement, aux environs de 3 moles d'acide par mole d'halogénure de ruthénium.

9. Procédé selon l'une des revendications 1 à 8 **caractérisé par le fait que** l'on prépare le complexe par mélange, dans un ordre quelconque, de l'halogénure de ruthénium (de préférence, le chlorure de ruthénium III) de l'halogénure d'étain (de préférence, le chlorure d'étain II) et de l'acide fort (de préférence, l'acide chlorhydrique).

10. Procédé selon la revendication 9 **caractérisé par le fait que** l'on porte le mélange réactionnel à une température allant de 20°C à 100°C, de préférence, entre 70°C et 90°C.

11. Procédé selon l'une des revendications 1 à 10 **caractérisé par le fait que** l'on ajoute un support sous forme de poudre, dans la solution de complexe obtenu dans l'une des revendications 1 à 10, que l'on hydrolyse le complexe par addition d'eau et que l'on sépare le solide obtenu, qu'on le malaxe et l'extrude ce qui conduit à un catalyseur mis en forme.

12. Procédé selon la revendication 11 **caractérisé par le fait que** la quantité d'eau mise en oeuvre représente de 1 à 100 fois le poids du complexe.

13. Procédé selon l'une des revendications 1 à 12 **caractérisé par le fait que** l'on dépose les métaux sur support en imprégnant ledit support avec la solution du complexe obtenu dans l'une des revendications 1 à 10.

14. Procédé selon la revendication 13 **caractérisé par** le lait que le support est sous forme de poudre, billes, granulés, extrudés et autres.

15. Procédé selon l'une des revendications 13 et 14 **caractérisé par le fait que** le support est choisi parmi les oxydes de métaux, de préférence, les oxydes d'aluminium, de silicium et/ou de zirconium, les charbons actifs et les résines.

16. Procédé selon l'une des revendications 13 à 15 **caractérisé par le fait que** la teneur en ruthénium du catalyseur supporté est choisie entre 0,1 et 20,0 % en poids, et encore plus préférentiellement, entre 0,5 et 3,0 % en poids.

17. Procédé selon l'une des revendications 1 à 14 **caractérisé par le fait que** la solution aqueuse d'imprégnation comprend le complexe de ruthénium et d'étain à raison de 1 % à 20 % en poids de ruthénium.

18. Procédé selon l'une des revendications 1 à 17 **caractérisé par le fait que** l'on réalise l'imprégnation en pulvérisant sur le support mis en mouvement, par exemple, par la rotation d'un drageoir, la solution aqueuse comprenant le complexe de ruthénium et d'étain.

19. Procédé selon l'une des revendications 1 à 18 **caractérisé par le fait que** l'on imprègne le support mis en forme en le plongeant dans la solution aqueuse dudit complexe.

20. Procédé selon l'une des revendications 1 à 18 **caractérisé par le fait que** l'on réalise l'imprégnation du support "à sec" à l'aide de la solution aqueuse comprenant le complexe de ruthénium et d'étain.

21. Procédé selon l'une des revendications 1 à 20 **caractérisé par le fait que** le support imprégné est soumis à un séchage effectué de préférence, à l'air à une température allant de la température ambiante jusqu'à 100°C.

22. Procédé selon l'une des revendications 1 à 21 **caractérisé par le fait que** l'on effectue la réduction dudit complexe en le mettant en contact avec de l'hydrogène.

23. Procédé selon la revendication 22 **caractérisé par le fait que** l'hydrogène peut être injecté à la pression atmosphérique ou sous une légère pression, par exemple de 0,5 à 10 bar, de préférence, entre 1 et 2 bar ou être dilué dans un gaz inerte tel que l'azote ou l'hélium.

24. Procédé selon l'une des revendications 1 à 23 **caractérisé par le fait que** la réaction de réduction est conduite à une température d'au moins 350°C, de préférence comprise entre 350°C et 600°C, et encore plus préférentiellement entre 400°C et 500°C.

25. Procédé selon l'une des revendications 1 à 24 **caractérisé par le fait que** la réaction de réduction est effectuée lors de l'utilisation du catalyseur.

26. Procédé selon l'une des revendications 1 à 25 **caractérisé par le fait que** le catalyseur obtenu présente une phase contenant le ruthénium et l'étain dans un rapport atomique Sn/Ru au moins égal à 2/3, avantageusement à 3/2, de préférence à 7/3.

27. Procédé selon la revendication 26 **caractérisé par le fait que** la phase contenant le ruthénium et l'étain présente un rapport atomique Sn/Ru au plus égal à 3, avantageusement à 5/2.

28. Procédé selon l'une des revendications 1 à 27 **caractérisé par le fait que** le catalyseur obtenu est supporté : la phase catalytique recouvrant au moins en partie ledit support contient au moins 50 %, avantageusement 80 %, de préférence au moins 90 % de ladite phase intermétallique.

29. Procédé selon l'une des revendications 1 à 28 **caractérisé par le fait que** que le catalyseur obtenu comprend du ruthénium dont au moins 90 %, avantageusement au moins 95 %, de préférence 98 % du ruthénium présent sur le support est sous la forme de ladite phase recouvrant ledit support.

## Patentansprüche

1. Verfahren zur Herstellung eines bimetallischen Ruthenium/Zinn-Katalysators, enthaltend einen Träger und eine metallische, den Träger zumindest teilweise bedeckende Phase, wobei der Träger zumindest teilweise ein intermetallisches Ruthenium-Zinn, das zumindest teilweise in Form der definierten Verbindung Ru₃Sn₇ vorliegt, enthält, **dadurch gekennzeichnet, daß** eine Reduktion eines Ruthenium-/Zinnkomplexes mit einer Elektronenvalenz von -4 und einer Koordinationszahl von 6 durchgeführt wird, wobei mindestens einer der Liganden ein Zinnhalogenid ist und die anderen Liganden gegebenenfalls aus einem Halogenatomen bestehen können.

2. Verfahren nach Anspruch 1, **dadurch gekennzeichnet, daß** der Komplex der folgenden Formel (A) entspricht:
[Ru(SnX₃)₆₋ₙXₙ]⁴⁻ (A)
wobei in der Formel (A) X einem Halogenatom, vorzugsweise einem Chlor-oder Bromatom, entspricht und n eine Zahl von 0 bis 2, vorzugsweise gleich 1, ist.

3. Verfahren nach Anspruch 2, **dadurch gekennzeichnet, daß** der Komplex der folgenden Formel (A) entspricht:
- [Ru(SnCl₃)₆]⁴⁻
- [Ru(SnCl₃)₅Cl]⁴⁻
- [Ru(SnCl₃)₄Cl₂]⁴⁻.

4. Verfahren nach einem der Ansprüche 1 bis 3, **dadurch gekennzeichnet, daß** der Komplex durch Reaktion eines Rutheniumhalogenids und eines Zinnhalogenids in Gegenwart einer Säure hergestellt wird.

5. Verfahren nach Anspruch 4, **dadurch gekennzeichnet, daß** das Rutheniumhalogenid ein Ruthenium(III)halogenid, vorzugsweise Rüthenium(III)chlorid, in wasserfreier oder hydratisierter Form ist und das Zinn(II)halogenid vorzugsweise Zinn(II)chlorid in wasserfreier oder hydratisierter Form ist.

6. Verfahren nach Anspruch 4 oder 5, **dadurch gekennzeichnet, daß** das Verhältnis der Molzahl des Rutheniumhalogenids und der Molzahl des Zinnhalogenids zwischen 0,10 und 0,5, vorzugsweise zwischen 0,15 und 0,35, variiert.

7. Verfahren nach einem der Ansprüche 4 bis 6, **dadurch gekennzeichnet, daß** die Säure jede starke Mineralsäure, vorzugsweise eine Wasserstoffsäure, ist, deren Halogenid identisch ist mit dem Halogenid, das in den Ruthenium-und Zinnsalzen verwendet wird.

8. Verfahren nach einem der Ansprüche 4 bis 7, **dadurch gekennzeichnet, daß** die Menge an verwendeter Säure insbesondere mindestens 1 mol Säure pro Mol Rutheniumhalogenid, vorzugsweise zwischen 1 und 5 mol Säure pro Mol Rutheniumhalogenid, weiter bevorzugt ungefähr 3 Mol Säure pro Mol Rutheniumhalogenid, beträgt.

9. Verfahren nach einem der Ansprüche 1 bis 8, **dadurch gekennzeichnet, daß** der Komplex durch Mischen des Rutheniumhalogenids (vorzugsweise des Ruthenium(III)chlorids), des Zinnhalogenids (vorzugsweise des Zinn(II)-chlorids) und der starken Säure (vorzugsweise der Salzsäure) in beliebiger Reihenfolge hergestellt wird.

10. Verfahren nach Anspruch 9, **dadurch gekennzeichnet, daß** die Reaktionsmischung auf eine Temperatur von 20 °C bis 100 °C, vorzugsweise zwischen 70 °C und 90 °C, gebracht wird.

11. Verfahren nach einem der Ansprüche 1 bis 10, **dadurch gekennzeichnet, daß** man einen Träger in Form eines Pulvers in die Lösung des Komplexes gibt, der nach einem der Ansprüche 1 bis 10 erhalten wird, daß der Komplex durch Zugabe von Wasser hydrolysiert wird und daß der erhaltene Feststoff abgetrennt wird, daß er geknetet und extrudiert wird, was zu einem geformten Katalysator führt.

12. Verfahren nach Anspruch 11, **dadurch gekennzeichnet, daß** die Menge des verwendeten Wassers das 1fache bis 100fache des Gewichts des Komplexes darstellt.

13. Verfahren nach einem der Ansprüche 1 bis 12, **dadurch gekennzeichnet, daß** die Metalle durch Imprägnieren des Trägers mit einer Lösung des in einem der Ansprüche 1 bis 10 erhaltenen Komplexes auf den Träger aufgebracht werden.

14. Verfahren nach Anspruch 13, **dadurch gekennzeichnet, daß** der Träger in Pulver-, Kugel-, Granulat-, extrudierter oder anderer Form vorliegt.

15. Verfahren nach einem der Ansprüche 13 und 14, **dadurch gekennzeichnet, daß** der Träger ausgewählt ist aus der Gruppe von Metalloxiden, vorzugsweise Aluminium-, Silicium- und/oder Zirconiumoxiden, Aktivkohlen und Harzen.

16. Verfahren nach einem der Ansprüche 13 bis 15, **dadurch gekennzeichnet, daß** der Gehalt an Ruthenium in dem Trägerkatalysator ausgewählt ist zwischen 0,1 und 20,0 Gew.-%, weiter bevorzugt zwischen 0,5 und 3,0 Gew.-%.

17. Verfahren nach einem der Ansprüche 1 bis 14, **dadurch gekennzeichnet, daß** die wäßrige Imprägnierlösung den Ruthenium-/Zinnkomplex in Mengen von 1 Gew.-% bis 20 Gew.-% Ruthenium enthält.

18. Verfahren nach einem der Ansprüche 1 bis 17, **dadurch gekennzeichnet, daß** das Imprägnieren durch Zersprühen der wäßrigen Lösung, die den Ruthenium-/Zinnkomplex enthält, über den Träger, der sich, z. B durch Rotation einer Dragiervorrichtung, in Bewegung befindet.

19. Verfahren nach einem der Ansprüche 1 bis 18, **dadurch gekennzeichnet, daß** der geformte Träger durch Eintauschen in die wäßrige Lösung des Komplexes imprägniert wird.

20. Verfahren nach einem der Ansprüche 1 bis 18, **dadurch gekennzeichnet, daß** die "trockene" Imprägnierung mit Hilfe der wäßrigen Lösung, die den Ruthenium-/Zinnkomplex enthält, durchgeführt wird.

21. Verfahren nach einem der Ansprüche 1 bis 20, **dadurch gekennzeichnet, daß** der imprägnierte Träger einer Trocknung unterzogen wird, vorzugsweise an der Luft und bei einer Temperatur von Umgebungstemperatur bis 100 °C.

22. Verfahren nach einem der Ansprüche 1 bis 21, **dadurch gekennzeichnet, daß** man die Reduktion des Komplexes durch Inkontaktbringen mit Wasserstoff durchführt.

23. Verfahren nach Anspruch 22, **dadurch gekennzeichnet, daß** der Wasserstoff bei Atmosphärendruck oder unter einem leichten Druck, z. B. 0,5 bis 10 bar, vorzugsweise zwischen 1 und 2 bar, oder verdünnt in einem Inertgas, wie Stickstoff oder Helium, eingeführt werden kann.

24. Verfahren nach einem der Ansprüche 1 bis 23, **dadurch gekennzeichnet, daß** die Reduktionsreaktion bei einer Temperatur von mindestens 350 °C, insbesondere zwischen 350 °C und 600 °C, vorzugsweise 400 °C und 500 °C, durchgeführt wird.

25. Verfahren nach einem der Ansprüche 1 bis 24, **dadurch gekennzeichnet, daß** die Reduktionsreaktion während der Verwendung des Katalysators durchgeführt wird.

26. Verfahren nach einem der Ansprüche 1 bis 25, **dadurch gekennzeichnet, daß** der erhaltene Katalysator eine Phase umfaßt, die Ruthenium und Zinn in einem Atomverhältnis Sn/Ru von mindestens gleich 2/3, insbesondere 3/2, vorzugsweise 7/3, enthält.

27. Verfahren nach Anspruch 26, **dadurch gekennzeichnet, daß** die Ruthenium und Zinn enthaltene Phase ein Atomverhältnis von höchstens gleich 3, vorzugsweise 5/2, enthält.

28. Verfahren nach einem der Ansprüche 1 bis 27, **dadurch gekennzeichnet, daß** der erhaltene Katalysator von der katalytischen Phase getragen wird, die zumindest teilweise den Träger bedeckt, der mindestens 50 %, insbesondere mindestens 80 %, vorzugsweise mindestens 90 %, der intermetallischen Phase enthält.

29. Verfahren nach einem der Ansprüche 1 bis 28, **dadurch gekennzeichnet, daß** der erhaltene Katalysator Ruthenium enthält, wovon mindestens 90 %, insbesondere mindestens 95 %, vorzugsweise 98 %, des Rutheniums, das auf dem Träger vorhanden ist, in Form der Phase vorliegt, die den Träger bedeckt.

## Claims

1. A process for the preparation of a bimetallic ruthenium/tin catalyst, comprising a support and a metallic phase covering at least part of said support comprising at least in part an intermetallic ruthenium/tin phase at least partially in the form of the described compound Ru₃Sn₇ **characterized in that** it consists in carrying out the reduction of a complex of ruthenium and tin having an electrovalency of -4 and a coordination number of 6, of which at least one of the ligands is a tin halide; the other ligands can be optionally constituted by a halogen atom.

2. A process according to claim 1, **characterized in that** the complex corresponds to the following formula (A):
[Ru(SnX₃)₆₋ₙXₙ]⁴- (A)
in which formula (A), X represents a halogen atom, preferably a chlorine or bromine atom, and n is a number from 0 to 2, and preferably equal to 1.

3. A process according to claim 2, **characterized in that** the complex corresponds to the following formula (A):
- [Ru(SnCl₃)₆]⁴⁻
- [Ru(SnCl₃)₅Cl]⁴⁻
-[Ru(SnCl₃)₄Cl₂]⁴⁻

4. A process according to one of claims 1 to 3, **characterized in that** the complex is prepared by reaction of a ruthenium halide and a tin halide in the presence of an acid.

5. A process according to claim 4, **characterized in that** the ruthenium halide is a ruthenium III halide in the anhydrous or hydrated form, preferably a ruthenium III chloride, and the tin II halide, in the anhydrous or hydrated form, is preferably tin II chloride.

6. A process according to one of claims 4 and 5, **characterized in that** the ratio between the number of moles of ruthenium halide and the number of moles of tin halide is between 0.10 and 0.5, and preferably between 0.15 and 0.35.

7. A process according to one of claims 4 to 6, **characterized in that** the acid is any strong mineral acid, preferably the hydrogen acid of which the halide is identical to the halide used in the ruthenium and tin salts.

8. A process according to one of claims 4 to 7, **characterized in that** the quantity of acid used is preferably at least 1 mole of acid per mole of ruthenium halide, more preferably between 1 and 5 moles of acid per mole of ruthenium halide and even more preferably in the region of 3 moles of acid per mole of ruthenium halide.

9. A process according to one of claims 1 to 8, **characterized in that** the complex is prepared by mixing, in any order, ruthenium halide (preferably ruthenium Ill chloride), tin halide (preferably tin II chloride) and strong acid (preferably hydrochloric acid).

10. A process according to claim 9, **characterized in that** the reaction mixture is raised to a temperature from 20°C to 100°C, preferably between 70°C and 90°C.

11. A process according to one of claims 1 to 10, **characterized in that** a support in the form of a powder is added to the solution of complex obtained in one of claims 1 to 10, the complex is hydrolyzed by adding water and the solid obtained is separated, mixed and extruded, which operation leads to a formed catalyst.

12. A process according to claim 11, **characterized in that** the quantity of water used represents 1 to 100 times the weight of the complex.

13. A process according to one of claims 1 to 12, **characterized in that** the metals are deposited on a support by impregnating said support with the solution of the complex obtained in one of claims 1 to 10.

14. A process according to claim 13, **characterized in that** the support is in the form of a powder, beads, granules, extrudates and others.

15. A process according to one of claims 13 and 14, **characterized in that** the support is chosen from metal oxides, preferably aluminium, silicon and/or zirconium oxides, activated carbons and resins.

16. A process according to one of claims 13 to 15, **characterized in that** the ruthenium content of the supported catalyst is between 0.1 and 20.0% by weight, and even more preferably between 0.5 and 3.0% by weight.

17. A process according to one of claims 1 to 14, **characterized in that** the aqueous impregnation solution contains the ruthenium and tin complex in a quantity of 1% to 20% by weight of ruthenium.

18. A process according to one of claims 1 to 17, **characterized in that** impregnation is carried out by spraying the aqueous solution containing the ruthenium and tin complex onto the support brought into motion, for example, by the rotation of a rotatable outer ring.

19. A process according to one of claims 1 to 18, **characterized in that** the formed support is impregnated by being immersed into the aqueous solution of the said complex.

20. A process according to one of claims 1 to 18, **characterized in that** impregnation of the support "in the dry state" is carried out using the aqueous solution containing the ruthenium and tin complex.

21. A process according to one of claims 1 to 20, **characterized in that** the impregnated support undergoes drying carried out preferably in the air at a temperature ranging from ambient temperature to 100°C.

22. A process according to one of claims 1 to 21 **characterized in that** the reduction of the said complex is carried out by bringing it into contact with hydrogen.

23. A process according to claim 22, **characterized in that** the hydrogen may be injected at atmospheric pressure or under a slight pressure, for example from 0.5 to 10 bar, preferably between 1 and 2 bar or it may be diluted in an inert gas such as nitrogen or helium.

24. A process according to one of claims 1 to 23, **characterized in that** the reduction reaction is carried out at a temperature of at least 350°C, preferably between 350°C and 600°C, and even more preferably between 400°C and 500°C.

25. A process according to one of claims 1 to 24, **characterized in that** the reduction reaction is carried out during the use of the catalyst.

26. A process according to one of claims 1 to 25 **characterized in that** the catalyst obtained presents a phase containing the ruthenium and tin in an atomic ratio Sn/Ru at least equal to 2/3, advantageously 3/2, preferably 7/3.

27. A process according to claim 26, **characterized in that** the phase containing the ruthenium and the tin has an atomic ratio Sn/Ru at most equal to 3, advantageously 5/2.

28. A process according to one of claims 1 to 27, **characterized in that** the catalyst obtained is supported: the catalytic phase covering at least in part the said support contains at least 50%, advantageously 80%, preferably at least 90% of the said intermetallic phase.

29. A process according to one of claims, 1 to 28, **characterized in that** the catalyst obtained contains ruthenium of which at least 90%, advantageously at least 95%, preferably 98% of the ruthenium present on the support is in the form of the said phase covering the said support.
